# FASCICULE DE BREVET EUROPEEN

(11) **EP 1 299 370 B1**
(45) Date de publication et mention de la délivrance du brevet: **09.06.2004**
(21) Numéro de dépôt: 01956503.5
(22) Date de dépôt: 26.06.2001
(51) Int. Cl.: C07D 301/12

(54) **PROCEDE CONTINU DE FABRICATION D'OXIRANNE**
KONTINUIERLICHES VERFAHREN ZUR HERSTELLUNG VON OXIRANEN
METHOD FOR CONTINUOUS OXIRANE PRODUCTION

(30) Priorité: 28.06.2000 FR 0008353
(43) Date de publication de la demande: 09.04.2003
(73) Titulaire: SOLVAY (Société Anonyme), 1050 Bruxelles (BE)
(72) Inventeur: BALTHASART, Dominique, B-1120 Bruxelles (BE)
(74) Mandataire: Vande Gucht, Anne
(86) Numéro de dépôt international: PCT/EP2001/007269
(87) Numéro de publication internationale: WO 2002/000634

(56) Documents cités:
- WO-A-99/32472
- WO-A-99/48883
- US-A- 3 632 482
- US-A- 4 070 253
- US-A- 5 274 138
- US-A- 5 354 430

## Description

La présente invention concerne un procédé de fabrication d'oxiranne par réaction entre une oléfine et un composé peroxydé en présence d'un catalyseur et d'un solvant. En particulier, elle concerne la fabrication d'oxyde de propylène par époxydation de propylène au moyen de peroxyde d'hydrogène en présence d'un catalyseur contenant du TS-1.

Il est connu de fabriquer de l'oxyde de propylène par réaction entre le propylène et un composé peroxydé en présence de TS-1. Par exemple, dans le brevet US 5,849,937 un tel procédé est réalisé dans plusieurs réacteurs disposés en série. Dans ce procédé connu, le milieu sortant du dernier réacteur peut être fractionné ou traité d'une autre manière pour récupérer l'époxyde. L'oléfine non convertie peut être recyclée.

Toutefois, dans ce procédé, le milieu sortant du dernier réacteur contient, outre l'oléfine non convertie, le solvant et éventuellement de l'eau. Ce procédé présente comme inconvénient qu'il est peu économique car il n'est pas envisagé de récupérer le solvant

La présente invention vise à remédier à cet inconvénient en fournissant un nouveau procédé qui permet de manière simple de récupérer le solvant, d'éliminer l'eau et de minimaliser la consommation de vapeur et/ou d'énergie.

A cet effet, l'invention concerne un procédé continu de fabrication d'oxiranne par réaction d'une oléfine avec un composé peroxydé en présence d'un catalyseur, d'un solvant et de l'eau dans une installation comprenant au moins un réacteur contenant le catalyseur et au moins deux colonnes à distiller, selon lequel :
- on introduit dans le réacteur l'oléfine, le solvant, le composé peroxydé et l'eau,
- on y effectue une époxydation de l'oléfine pour former l'oxiranne,
- on soutire du réacteur un milieu comprenant l'oxiranne formé, l'oléfine non convertie, le solvant, le composé peroxydé non consommé, l'eau et éventuellement des sous-produits,
- on introduit le milieu dans une colonne à distiller (A),
- on recueille en tête de la colonne (A) un mélange contenant la majeure partie de l'oxiranne formé et de l'oléfine non convertie, du solvant, de l'eau et éventuellement des sous-produits,
- on introduit le mélange dans un condenseur pour éliminer une partie de l'oléfine non convertie,
- on recueille le mélange appauvri en oléfine non convertie sous forme liquide,
- on introduit le mélange liquide dans une deuxième colonne à distiller (B),
- on recueille en pied de la colonne (B) un mélange de solvant et d'eau que l'on recycle dans le réacteur, et
- on recueille en tête de la colonne (B) un milieu à base d'oxiranne.

Une des caractéristiques essentielles de la présente invention réside dans la possibilité de recycler directement dans le réacteur sans traitement de purification et/ou d'élimination de l'eau, le milieu recueilli en pied de la colonne (B). Ceci permet de récupérer le solvant de manière simple sans traitement de purification, tout en gardant une bonne productivité.

La distillation dans la colonne (A) a pour fonction d'éliminer le plus vite possible l'oxiranne dès sa formation dans le milieu réactionnel pour éviter qu'il entre en contact avec les autres constituants du milieu réactionnel et pour éviter que des sous-produits soient ainsi formés. La demanderesse a en effet constaté que, lorsque l'on fabrique de l'oxyde de propylène au moyen d'une solution aqueuse de peroxyde d'hydrogène à titre de composé peroxydé et du méthanol à titre de solvant, des sous-produits sont formés par réaction entre l'oxyde de propylène et l'eau ou le méthanol, et notamment le propylène glycol et des méthoxypropanols de formule CH₃-CHOH-CH₂-OCH₃ et CH₃-CH(OCH₃)-CH₂OH. Lorsque l'épichlorhydrine est fabriquée, des sous-produits sont formés par réaction entre l'épichlorhydrine et l'eau ou le méthanol, et notamment le 1-chloropropanediol et des chlorométhoxypropanols de formule ClCH₂-CHOH-CH₂-OCH₃ et Cl-CH₂-CH(OCH₃)-CH₂OH. La formation de sous-produits réduit la sélectivité du procédé et dès lors son rendement.

La séparation dans la colonne (A) est effectuée dans une colonne à distiller séparée et distincte du réacteur d'époxydation. Puisque le catalyseur ne quitte pas le réacteur d'époxydation, la distillation est donc effectuée en l'absence du catalyseur d'époxydation afin d'éviter le contact entre l'oxiranne formé et le catalyseur d'époxydation car celui-ci favorise la formation des sous-produits.

Le mélange recueilli en tête de la colonne (A) contient généralement :
- de l'oxiranne en une concentration de 30 à 90 % en poids, le plus souvent de 45 à 75 % en poids;
- de l'oléfine non convertie en une concentration de 2 à 20 % en poids, le plus souvent de 5 à 15 % en poids;
- du solvant en une concentration de 5 à 40 % en poids, le plus souvent de 10 à 25% en poids;
- de l'eau en une concentration de moins de 5 % en poids, le plus souvent de moins de 2 % en poids.

Le mélange recueilli en pied de la colonne (A) contient généralement :
- du solvant en une concentration de 25 à 99 % en poids, en particulier de 50 à 95 % en poids;
- de l'oléfine non convertie en une concentration de moins de 1 % en poids, en particulier de moins de 0,1 % en poids;
- du composé peroxydé non consommé en une concentration de 0,1 à 10 % en poids, en particulier de 0,2 à 6 % en poids;
- de l'eau en une concentration de 5 à 60 % en poids, en particulier de 20 à 50 % en poids;
- des sous-produits en une concentration de 0,1 à 10 % en poids, en particulier de 0,2 à 6 % en poids.

Le condenseur disposé en aval de la colonne (A) a pour fonction d'éliminer une partie de l'oléfine non convertie présente dans le milieu entrant dans le condenseur, et de récupérer un mélange appauvri en oléfine non convertie sous forme liquide. Le mélange liquide sortant du condenseur ne contient généralement pas plus que 2 % en poids d'oléfine non convertie, en particulier pas plus que 0,5 % en poids.

La distillation dans la colonne (B) a pour fonction de séparer le solvant et l'eau du milieu entrant la colonne (B). Après séparation, le milieu contenant l'oxiranne, que l'on recueille en tête de la colonne (B), ne contient généralement pas plus que 10 % en poids de solvant, en particulier pas plus que 5 % en poids. Il ne contient habituellement pas plus que 0,5 % en poids d'eau, en particulier pas plus que 0,1 % en poids.

Le mélange de solvant et d'eau recueilli en pied de la colonne (B) contient généralement :
- de 25 à 99 % en poids de solvant, en particulier de 50 à 95 % en poids; et
- de 0 à 20 % en poids d'eau, en particulier de 0 à 10 % en poids.

Les conditions dans lesquelles sont réalisées chaque distillation (dans les colonnes (A) et (B)) dépendent de la nature de l'oxiranne (et notamment de sa température d'ébullition), de sa concentration dans le milieu introduit dans la colonne à distiller, de la nature des autres constituants du milieu, de leur température d'ébullition et du rendement souhaité de la distillation.

Chaque distillation est généralement réalisée à une température supérieure ou égale à 10°C, de préférence supérieure ou égale à 40°C, les valeurs supérieures ou égales à 50°C étant recommandées. La température est habituellement inférieure ou égale à 125°C, le plus souvent inférieure ou égale à 110°C, les valeurs inférieures ou égales à 100°C étant préférées.

Chaque distillation est couramment effectuée à une pression supérieure ou égale à 0,2 bar, de préférence supérieure ou égale à 0,5 bar, les valeurs supérieures ou égales à 1 bar étant les plus courantes. La pression est en général inférieure ou égale à 5 bar, en particulier inférieure ou égale à 3 bar, les valeurs inférieures ou égales à 2 bar étant tout particulièrement recommandées. Dans la présente description, toute référence à la pression de la distillation correspond à la pression absolue mesurée en tête de la colonne de distillation.

Chaque colonne de distillation utilisable dans le procédé selon l'invention est connue en elle-même. On peut utiliser, par exemple, une colonne à plateaux conventionnels ou une colonne à plateaux de type "dual-flow" ou encore une colonne à empilage en vrac ou structuré.

Chaque distillation permet de séparer la majeure partie de l'oxiranne formé du milieu réactionnel d'époxydation. Cette majeure partie est en général supérieure ou égale à 80 % de la quantité d'oxiranne formé dans le premier réacteur. Elle est le plus souvent supérieure ou égale à 90 %. Habituellement, elle est inférieure ou égale à 99,9 %. En particulier inférieure ou égale à 99 %.

Le nombre de plateaux théoriques dans chaque colonne à distiller est généralement supérieur ou égal à 20, plus spécialement supérieur ou égal à 40. Un nombre inférieur ou égal à 80 donne de bons résultats. Un nombre inférieur ou égal à 60 est recommandé.

Le taux de reflux molaire (qui correspond au débit molaire de liquide renvoyé en tête de colonne ramené à l'ensemble du distillat - vapeur plus liquide - prélevé en tête de colonne) dans chaque colonne à distiller est habituellement supérieur ou égal à 0,5, de préférence supérieur ou égal à 0,8. Ce taux est couramment inférieur ou égal à 5, le plus souvent inférieur ou égal à 2,5.

Dans une première forme de réalisation particulière du procédé selon l'invention, on introduit le milieu recueilli en tête de la colonne (B) dans une troisième colonne à distiller (C) et on recueille en tête de la colonne (C) de l'oléfine non convertie. Celle-ci peut avantageusement être recyclée dans le réacteur.

La distillation dans la colonne (C) a pour fonction d'éliminer l'oléfine non convertie du milieu entrant dans la colonne (C) et donc de purifier le milieu contenant l'oxiranne en oléfine non convertie. Lorsque l'oléfine introduite dans le réacteur contient des impuretés, en général légères, celles-ci sont également éliminées dans la colonne (C). On peut citer à titre d'exemple des sources de propylène qui contiennent jusque 10 % en poids de propane.

La distillation dans la colonne (C) peut être effectuée dans les conditions précisées plus haut pour les colonnes (A) et (B).

Le milieu recueilli en tête de la colonne (C) contient généralement :
- de 30 à 90 % en poids d'oléfine, en particulier de 40 à 70 % en poids; et
- de 10 à 70 % en poids d'impuretés introduites avec l'oléfine dans le réacteur, en particulier de 30 à 60 % en poids.

Le milieu recueilli en pied de la colonne (C) ne contient généralement pas plus que 0,05 % en poids d'oléfine non convertie, en particulier pas plus que 0,01 % en poids. Il ne contient généralement pas plus que 0,1 % en poids d'impuretés introduites avec l'oléfine, en particulier pas plus que 0,02 % en poids.

Dans une deuxième forme de réalisation du procédé selon l'invention, on introduit le milieu recueilli en pied de la colonne (C) dans une quatrième colonne à distiller (D) et on recueille en tête de la colonne (D) l'oxiranne formé. Le milieu recueilli en pied de la colonne (D), qui contient des sous-produits, peut avantageusement être recyclé dans la colonne (B). Ce recyclage permet d'éliminer de l'oxiranne les sous-produits formés dans la colonne (C) par réaction de l'oxiranne avec le solvant (en particulier l'alcool) et l'eau ou par corrosion tout en permettant la récupération de l'oxiranne entraîné.

La distillation dans la colonne (D) a pour fonction d'éliminer les impuretés lourdes telles que les sous-produits décrits plus haut du milieu entrant dans la colonne (D), les sous-produits formés dans la colonne (D) même et les produits de corrosion, et donc de purifier le milieu contenant l'oxiranne en impuretés lourdes.

La distillation dans la colonne (D) peut être effectuée dans les conditions précisées plus haut pour les colonnes (A) et (B).

Le produit final recueilli en tête de la colonne (D) ne contient généralement pas plus que 0,1 % en poids d'impuretés lourdes, en particulier pas plus que 0,01 % en poids. Il contient généralement au moins 95 % en poids d'oxiranne, en particulier au moins 98 %, de préférence au moins 99,5 % en poids. On peut atteindre une pureté de 99,9 voire 99,99 % en poids.

Dans une troisième forme de réalisation du procédé selon l'invention, le réacteur est constitué de deux réacteurs disposés en parallèle, les deux réacteurs pouvant opérer simultanément ou alternativement. Cette troisième forme de réalisation présente l'avantage de pouvoir fabriquer de l'oxiranne en continu avec un rendement relativement constant bien que l'activité catalytique du catalyseur d'époxydation chute avec le temps. Cette forme de réalisation permet en effet d'arrêter un des deux réacteurs pour régénérer ou remplacer le catalyseur y présent pendant que l'autre réacteur continue de fabriquer.

Dans une quatrième forme de réalisation du procédé selon l'invention, le milieu soutiré du réacteur est d'abord soumis à une détente avant de l'introduire dans la colonne (A). Cette forme convient particulièrement bien lorsque l'époxydation est réalisée sous pression ou en présence d'un composé gazeux. Ce composé gazeux peut être l'oléfine-même (par exemple le propylène) ou un gaz inerte que l'on introduit dans le milieu réactionnel d'époxydation pour permettre d'entraîner l'oxiranne et de le sortir du réacteur, comme décrit dans la demande de brevet WO 99/48883 de la demanderesse.

Dans une cinquième forme de réalisation du procédé selon l'invention, le catalyseur est présent dans le réacteur sous forme de particules dont une partie au moins se trouve à l'état fluidisé, comme décrit dans la demande de brevet de la demanderesse déposée le même jour que la présente demande de brevet et intitulée "Procédé de fabrication d'oxiranne en présence d'un catalyseur sous forme de particules" (dont le contenu est incorporé par référence). Cette forme de réalisation permet d'obtenir une dispersion homogène du catalyseur dans le milieu réactionnel d'époxydation, un bon échange thermique et donc un contrôle aisé de la température de réaction. Dans cette forme, il est recommandé que le milieu sortant du réacteur passe à travers un filtre avant d'être introduit dans la colonne (A) ou, le cas échéant, avant d'être soumis à la détente.

Dans une sixième forme de réalisation préférée, l'installation comporte au moins deux réacteurs disposés en série, dont chacun contient une partie du catalyseur, et au moins trois colonnes à distiller, et selon lequel:
- on introduit dans un premier réacteur une première partie de l'oléfine, le solvant et le composé peroxydé,
- on y effectue une époxydation de la première partie de l'oléfine pour former une première partie de l'oxiranne,
- on soutire de ce premier réacteur un milieu comprenant la première partie d'oxiranne formé, le solvant, l'oléfine non convertie, le composé peroxydé non consommé et éventuellement des sous-produits,
- on introduit le milieu dans une première colonne à distiller (A),
- on recueille en tête de la colonne (A) un premier mélange contenant la majeure partie de l'oxiranne formé et de l'oléfine non convertie, du solvant de l'eau et éventuellement des sous-produits,
- on recueille en pied de la colonne (A) un premier milieu appauvri en oxiranne contenant du solvant, de l'oléfine non convertie, du composé peroxydé non consommé et éventuellement des sous-produits,
- on introduit dans un deuxième réacteur le premier milieu appauvri en oxiranne, une deuxième partie de l'oléfine et éventuellement une deuxième partie du composé peroxydé,
- on y effectue une époxydation de la deuxième partie de l'oléfine pour former une deuxième partie de l'oxiranne,
- on soutire de ce deuxième réacteur un milieu comprenant la deuxième partie d'oxiranne formé, du solvant, de l'oléfine non convertie, du composé peroxydé non consommé et éventuellement des sous-produits,
- on introduit le milieu dans une autre colonne à distiller (A'),
- on recueille en tête de la colonne (A') un deuxième mélange contenant la majeure partie de l'oxiranne formé et de l'oléfine non convertie, du solvant, de l'eau et éventuellement des sous-produits,
- on recueille en pied de la colonne (A') un deuxième milieu appauvri en oxiranne contenant du solvant, de l'oléfine non convertie, du composé peroxydé non consommé et éventuellement des sous-produits,
- on introduit chacun des premier et deuxième mélanges contenant la majeure partie de l'oxiranne formé et l'oléfine non convertie dans un condenseur pour séparer une partie de l'oléfine non convertie,
- on soutire de chaque condenseur un mélange appauvri en oléfine non convertie sous forme liquide,
- on introduit le mélange liquide dans une deuxième colonne à distiller (B),
- on recueille en pied de la colonne (B) un mélange de solvant et d'eau que l'on recycle dans le premier réacteur, et
- on recueille en tête de la colonne (B) un milieu à base d'oxiranne.

Dans cette sixième forme de réalisation du procédé selon l'invention, il peut être avantageux d'introduire l'entièreté du composé peroxydé dans le premier réacteur, comme décrit dans la demande de brevet de la demanderesse déposée le même jour que la présente demande de brevet et intitulée "Procédé de fabrication d'oxiranne au moyen d'un composé peroxydé" (dont le contenu est incorporé par référence). Le ou les réacteurs ultérieurs ne sont donc pas alimentés en composé peroxydé frais mais seulement avec le composé peroxydé qui est présent dans le milieu provenant du réacteur précédent et qui n'a pas été consommé dans ce réacteur précédent. Le fait de ne pas ajouter du composé peroxydé dans le(s) réacteur(s) ultérieur(s) permet de consommer 100 % de la quantité totale de composé peroxydé mise en oeuvre sans pour autant diminuer la vitesse de réaction par rapport à un procédé utilisant la même quantité totale de composé peroxydé mais dans lequel chaque réacteur est alimenté en composé peroxydé frais.

Dans cette sixième forme de réalisation du procédé selon l'invention, il peut être avantageux de recycler une fraction du milieu produit en pied de l'une des colonnes (A) et (A') vers le réacteur directement en amont. Ceci permet de maintenir constante la vitesse de circulation du milieu réactionnel dans le réacteur et de ne pas perturber l'écoulement. Cette pratique est particulièrement avantageuse lorsque le catalyseur est mis en oeuvre à l'état fluidisé.

Dans cette sixième forme de réalisation, l'installation peut évidemment comprendre plus de deux réacteurs connectés en série. L'installation comporte de préférence trois réacteurs disposés en série. Dans ce cas:
- on introduit, dans un troisième réacteur, une troisième partie de l'oléfine, le deuxième milieu recueilli en pied de la colonne (A'), et éventuellement une troisième partie du composé peroxydé,
- on y effectue une époxydation de la troisième partie d'oléfine pour former une troisième partie de l'oxiranne,
- on soutire de ce troisième réacteur un milieu comprenant la troisième partie d'oxiranne, le solvant, l'oléfine non convertie, éventuellement le composé peroxydé non consommé et éventuellement des sous-produits,
- on introduit ce milieu dans encore une autre colonne à distiller (A"),
- on recueille en tête de la colonne (A") un milieu que l'on recycle, après condensation, dans le premier réacteur,
- on recueille en pied de la colonne (A") un effluent aqueux

Les distillations dans les colonnes (A') et (A") ont la même fonction que celle dans la colonne (A) et peuvent être effectuées comme dans la colonne (A).

Dans cette sixième forme de réalisation, chaque réacteur est alimenté en oléfine. Le composé peroxydé et le solvant sont introduits dans le premier réacteur. Du composé peroxydé frais peut également être introduit dans un ou plusieurs des réacteurs ultérieurs. Chaque réacteur contient une partie du catalyseur qui ne quitte pas ce réacteur. Lorsque le catalyseur est présent sous la forme d'un lit fixe, il n'est en général pas nécessaire de prendre des précautions pour maintenir le catalyseur dans le réacteur. Par contre, lorsque le catalyseur est présent sous la forme de particules dont une partie au moins est à l'état fluidisé, il est recommandé de prévoir une zone de dégagement surmontant le lit fluide pour arrêter les particules de catalyseur qui sont en mouvement et/ou de prévoir un filtre à la sortie du réacteur.

Dans cette sixième forme de réalisation, on utilise de préférence des réacteurs de dimension identique. Ceci permet de permuter la fonction des réacteurs lorsque le catalyseur désactivé d'un réacteur est remplacé par du catalyseur frais ou régénéré sans perturber le fonctionnement de l'installation (fonctionnement dit "en carrousel").

Les formes de réalisation décrites ci-avant peuvent être combinées entre elles.

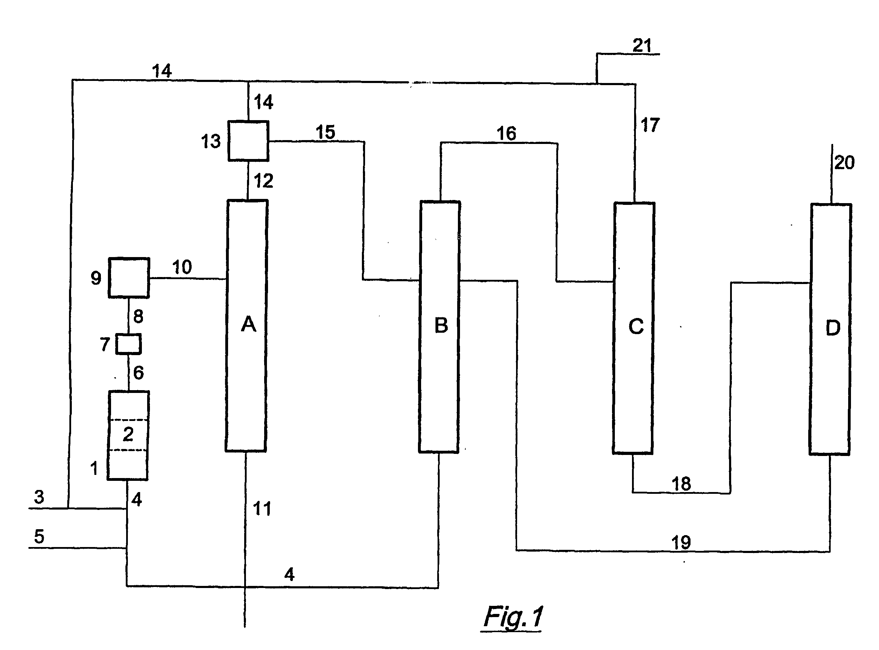

Une forme de réalisation qui convient bien est schématisée dans la figure 1. Dans cette forme, le réacteur 1 contient le catalyseur, de préférence en lit fluide 2. Le réacteur 1 est alimenté en oléfine par le conduit 3 et ensuite par le conduit 4, en composé peroxydé par le conduit 5 en ensuite par le conduit 4, et en solvant par le conduit 4 provenant d'une autre partie de l'installation qui est décrite plus loin. Dans le réacteur, l'oléfine réagit avec le composé peroxydé en présence du catalyseur pour former l'oxiranne. Le milieu sortant du réacteur 1 via le conduit 6 contient le solvant, l'oxiranne, le composé peroxydé non consommé, l'oléfine non convertie et éventuellement des sous-produits. Ce milieu passe à travers un filtre 7, et est envoyé via le conduit 8 dans le récipient 9 où il est soumis à une détente. Le milieu est ensuite transporté via le conduit 10 dans une colonne à distiller (A). En pied de la colonne à distiller (A), on recueille un milieu contenant du solvant, du composé peroxydé non consommé et éventuellement des sous-produits. Ce milieu, dont une partie peut être recyclée dans le réacteur via les conduits 11 et 4, est purgé pour évacuer un effluent aqueux qui contient les impuretés. En tête de la colonne (A), on récupère un mélange d'oxiranne et d'oléfine non convertie. Ce mélange est envoyé via le conduit 12 dans un condenseur 13 qui sépare l'oxiranne de l'oléfine non convertie. L'oléfine non convertie est recyclée dans le réacteur 1 via les conduits 14, 3 et 4. Une purge 21 permet d'évacuer les impuretés gazeuses. Le mélange liquide appauvri en oléfine et recueilli du condenseur est envoyé via le conduit 15 dans une colonne à distiller (B). Le mélange de solvant et d'eau recueilli en pied de la colonne (B) est recyclé via le conduit 4 dans le réacteur 1. Le milieu recueilli en tête de la colonne (B), qui contient l'oxiranne et qui est purifié en solvant et eau, est envoyé via le conduit 16 dans une colonne à distiller (C). En tête de la colonne (C) on recueille l'oléfine non convertie que l'on recycle via les conduits 17, 14, 3 et 4 dans le réacteur 1. Le milieu recueilli en pied de la colonne (C), qui contient l'oxiranne et qui est purifié en solvant, eau et oléfine non convertie, est envoyé via le conduit 18 dans une colonne à distiller (D). En pied de la colonne (D), on recueille les impuretés lourdes que l'on recycle via le conduit 19 dans la colonne (B). En tête de la colonne (D) on recueille via le conduit 20 l'oxiranne purifié en solvant, eau, oléfine non convertie, impuretés légères et lourdes.

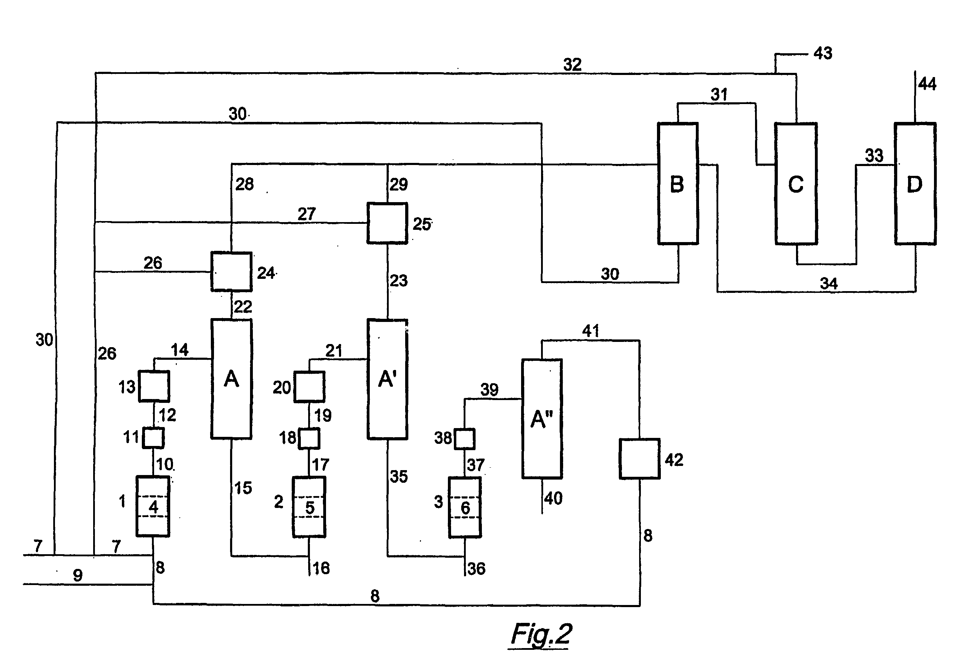

Une forme de réalisation particulièrement préférée est schématisée dans la figure 2. On y utilise trois réacteurs disposés en série. Dans cette forme, les réacteurs 1, 2 et 3 contiennent chacun une partie du catalyseur, de préférence en lit fluide 4, 5 et 6. Le réacteur 1 est alimenté en une première partie de l'oléfine par le conduit 7 et ensuite par le conduit 8, en composé peroxydé par le conduit 9 et ensuite par le conduit 8, et en solvant par le conduit 8 provenant d'une autre partie de l'installation qui est décrite plus loin. Dans le réacteur 1, la première partie de l'oléfine réagit avec le composé peroxyde en présence du catalyseur pour former une première partie de l'oxiranne. Le milieu sortant du réacteur 1 via le conduit 10 contient le solvant, la première partie de l'oxiranne, le composé peroxydé non consommé, l'oléfine non convertie et éventuellement des sous-produits. Ce milieu passe à travers un filtre 11, et est envoyé via le conduit 12 dans le récipient 13 où il est soumis à une détente. Le milieu est ensuite transporté via le conduit 14 dans une colonne à distiller (A). En pied de la colonne à distiller (A), on recueille un milieu contenant du solvant, du composé peroxydé non consommé et éventuellement une partie de l'oléfine non convertie. Ce milieu est transporté via le conduit 15 dans le deuxième réacteur 2. Le réacteur 2 est alimenté en une deuxième partie de l'oléfine via le conduit 16. Dans le réacteur 2, la deuxième partie de l'oléfine réagit avec le composé peroxydé non consommé provenant du premier réacteur en présence du catalyseur 5 pour former une deuxième partie de l'oxiranne. Le milieu sortant du réacteur 2 via le conduit 17 contient alors du solvant, la deuxième partie de l'oxiranne, de l'oléfine non convertie et du composé peroxydé non consommé dans le réacteur 2. Ce milieu passe à travers un filtre 18, et est envoyé via le conduit 19 dans le récipient 20 où il est soumis à une détente. Le milieu est ensuite transporté via le conduit 21 dans une colonne à distiller (A'). En tête de chaque colonne (A) et (A'), on récupère deux mélanges d'oxiranne et d'oléfine non convertie. Ces mélanges sont envoyés respectivement via les conduits 22 et 23 dans des condenseurs 24 et 25 qui séparent l'oxiranne de l'oléfine non convertie. L'oléfine non convertie est recyclée dans le réacteur 1 via les conduits 26, 7 et 8 pour celle provenant de la colonne (A) et via les conduits 27, 26, 7 et 8 pour celle provenant de la colonne (A'). Une purge 43 permet d'évacuer les impuretés gazeuses. Les mélanges liquides appauvris en oléfine et recueillis des condenseurs sont envoyés via les conduits 28 et 29 dans une colonne à distiller (B). Le mélange de solvant et d'eau recueilli en pied de la colonne (B) est recyclé via les conduits 3 0, 7 et 8 dans le réacteur 1. Le milieu recueilli en tête de la colonne (B), qui contient l'oxiranne et qui est purifié en solvant et eau, est envoyé via le conduit 31 dans une colonne à distiller (C). En tête de la colonne (C) on recueille l'oléfine non convertie que l'on recycle via les conduits 32, 26, 7 et 8 dans le réacteur 1. Le milieu recueilli en pied de la colonne (C), qui contient l'oxiranne et qui est purifié en solvant, eau et oléfine non convertie, est envoyé via le conduit 33 dans une colonne à distiller (D). En pied de la colonne (D), on recueille les impuretés lourdes que l'on recycle via le conduit 34 dans la colonne (B). En tête de la colonne (D) on recueille via le conduit 44 l'oxiranne purifié en solvant, eau, oléfine non convertie, impuretés légères et lourdes. En pied de la colonne (A'), on recueille un milieu contenant du solvant, du composé peroxydé non consommé dans le réacteur 2 et éventuellement une partie de l'oléfine non convertie. Ce milieu est transporté via le conduit 35 dans le troisième réacteur 3. Le réacteur 3 est alimenté en une troisième partie de l'oléfine via le conduit 36. Dans le réacteur 3, la troisième partie de l'oléfine réagit avec le composé peroxydé non consommé provenant du deuxième réacteur en présence du catalyseur 6 pour former une troisième partie de l'oxiranne. Les conditions dans le réacteur 3 sont telles que la totalité du composé peroxydé non encore consommé dans le deuxième réacteur soit consommée dans le réacteur 3. Le milieu sortant du réacteur 3 via le conduit 37 contient alors du solvant, la troisième partie de l'oxiranne, de l'oléfine non convertie et éventuellement des sous-produits. Ce milieu passe à travers un filtre 38, et est envoyé via le conduit 39 dans une colonne à distiller (A"). En pied de la colonne (A") on recueille via le conduit 40 un effluent aqueux. Le milieu recueilli en tête de la colonne (A") est envoyée via le conduit 41 dans un condenseur 42 et est ensuite recyclé via le conduit 8 dans le premier réacteur 1.

Le catalyseur utilisé dans le procédé selon l'invention contient généralement une zéolite comme élément actif, et de manière préférée, une zéolite au titane. Par zéolite au titane, on entend désigner un solide contenant de la silice qui présente une structure cristalline microporeuse de type zéolite et dans laquelle plusieurs atomes de silicium sont remplacés par des atomes de titane. La zéolite au titane présente avantageusement une structure cristalline de type ZSM-5, ZSM-11, ZSM-12, MCM-41, ZSM-48. Elle peut aussi présenter une structure cristalline de type zéolite bèta, de préférence exempte d'aluminium. Les zéolites présentant une bande d'absorption infrarouge à environ 950-960 cm-1 conviennent bien. Les zéolites au titane de type silicalite sont préférées. Celles répondant à la formule xTiO2(1-x)SiO2 dans laquelle x est de 0,0001 à 0,5 de préférence de 0,001 à 0,05, sont performantes. Des matériaux de ce type, connus sous le nom de TS-1, présentent une structure zéolitique cristalline microporeuse analogue à celle de la zéolite ZSM-5.

Le catalyseur utilisé dans le procédé selon l'invention se présente avantageusement sous la forme de particules obtenues par extrusion comme décrit dans la demande de brevet WO 99/28029 de la demanderesse, ou par un procédé en spray comme décrit dans la demande de brevet WO 99/24164 de la demanderesse. Le contenu de ces deux demandes de brevet est incorporé par référence dans celle-ci.

Le solvant mis en oeuvre dans le procédé selon l'invention peut être choisi parmi les alcools aliphatiques saturés, linéaires ou branchés. Le solvant alcoolique contient généralement jusqu'à 10 atomes de carbone, de préférence de 1 à 6 atomes de carbone. On peut citer à titre d'exemples le méthanol et l'éthanol. Le méthanol est préféré.

La quantité de solvant mise en oeuvre dans le premier réacteur est généralement d'au moins 25 % en poids du milieu réactionnel liquide présent dans le premier réacteur, en particulier d'au moins 40 % en poids, par exemple d'au moins 50 % en poids. Cette quantité ne dépasse habituellement pas 99 % en poids, en particulier pas 95 % en poids.

Le rapport molaire entre les quantités d'oléfine et de composé peroxydé engagées dans le procédé selon l'invention est généralement d'au moins 0.1, en particulier d'au moins 0,2, et de préférence d'au moins 0,5. Ce rapport molaire est le plus souvent d'au plus 100, en particulier d'au plus 50 et de préférence d'au plus 25.

Dans le procédé selon l'invention, le composé peroxydé est généralement mis en oeuvre dans le premier réacteur en une quantité d'au moins 0,005 mole par heure et par gramme de catalyseur présent dans le premier réacteur, en particulier, d'au moins 0,01 mole. La quantité de composé peroxydé est habituellement inférieure ou égale à 25 moles et, en particulier, inférieure ou égale à 10 moles. Une préférence est montrée pour une quantité de composé peroxydé supérieure ou égale à 0,03 mole et inférieure ou égale à 2,5 mole.

Dans le procédé selon l'invention le composé peroxydé est avantageusement mis en oeuvre sous forme d'une solution aqueuse. En général, la solution aqueuse contient au moins 2 % en poids de composé peroxydé, en particulier au moins 5 % en poids. Elle contient le plus souvent au maximum 90 % en poids de composé peroxydé, en particulier 70 % en poids. Une concentration de 30 à 40 % en poids, par exemple d'environ 36 % en poids, convient particulièrement bien.

La température de la réaction entre l'oléfine et le composé peroxydé peut varier de 10 à 125 °C. Dans une variante avantageuse telle que décrite dans la demande de brevet EP99/08703 de la demanderesse, elle est supérieure à 35 °C pour remédier à la désactivation progressive du catalyseur. La température peut être supérieure ou égale à 40 °C et de préférence supérieure ou égale à 45 °C. Une température supérieure ou égale à 50 °C est tout particulièrement préférée. La température de réaction est de préférence inférieure à 100 °C.

Dans le procédé selon l'invention, la réaction entre l'oléfine et le composé peroxydé peut avoir lieu à pression atmosphérique. Elle peut également se dérouler sous pression. Généralement, cette pression n'excède pas 40 bar. Une pression de 20 bar convient bien en pratique.

Les composés peroxydés qui peuvent être utilisés dans le procédé selon l'invention sont les composés peroxydés contenant une ou plusieurs fonctions peroxyde (-OOH) qui peuvent libérer de l'oxygène actif et capables d'effectuer une époxydation. Le peroxyde d'hydrogène et les composés peroxydés qui peuvent produire du peroxyde d'hydrogène dans les conditions de la réaction d'époxydation conviennent bien. Le peroxyde d'hydrogène est préféré.

Lorsqu'on utilise du peroxyde d'hydrogène, il peut être intéressant de mettre en oeuvre dans le procédé selon l'invention une solution aqueuse de peroxyde d'hydrogène à l'état brut, c'est-à-dire non épurée. Par exemple, on peut mettre en oeuvre une solution obtenue par simple extraction avec de l'eau substantiellement pure du mélange issu de l'oxydation d'au moins une alkylanthrahydroquinone (procédé appelé "procédé AO auto-oxydation") sans traitement ultérieur de lavage et/ou de purification. Ces solutions brutes de peroxyde d'hydrogène contiennent généralement de 0,001 à 10 g/l d'impuretés organiques exprimées en COT (Carbone Organique Total). Elles contiennent habituellement des cations métalliques (tels que des métaux alcalins ou alcalino-terreux, comme le sodium) et des anions (tels que les phosphates, nitrates) en des teneurs de 0,01 à 10 g/l.

Dans une autre variante du procédé, on peut mettre en oeuvre une solution de peroxyde d'hydrogène produite par synthèse directe à partir d'oxygène et d'hydrogène en présence de méthanol.

Lorsqu'on utilise du peroxyde d'hydrogène, une faible décomposition de celui-ci en eau et en oxygène peut être rencontrée au cours du procédé. Cet oxygène se retrouve dans l'oléfine non convertie. Afin d'éviter tout risque d'explosion, il est recommandé de traiter la navette gazeuse contenant l'oléfine non convertie et l'oxygène pour éliminer l'oxygène. Ce traitement peut être réalisé par exemple par l'utilisation d'une pervaporation au moyen de membranes ou encore par un traitement chimique par exemple au moyen de sulfite.

L'oxiranne qui peut être préparé par le procédé selon l'invention est un composé organique comprenant un groupement répondant à la formule générale :

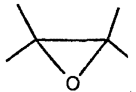

L'oxiranne contient généralement de 2 à 10 atomes de carbone, de préférence de 3 à 6 atomes de carbone. Les oxirannes qui peuvent être préparés de manière avantageuse par le procédé selon l'invention sont le 1,2-époxypropane et le 1,2-époxy-3-chloropropane. L'oxiranne préféré est le 1,2-époxypropane.

Les oléfines qui conviennent bien dans le procédé selon l'invention contiennent généralement de 2 à 10 atomes de carbone et de manière préférée, 3 à 6 atomes de carbone. Le propylène, le butylène et le chlorure d'allyle conviennent bien. Le propylène et le chlorure d'allyle sont préférés et tout particulièrement le propylène.

Dans le procédé selon l'invention il peut s'avérer intéressant de contrôler le pH de la phase liquide. Par exemple, il peut être intéressant de maintenir le pH de la phase liquide lors de la réaction entre l'oléfine et le composé peroxydé à une valeur de 4,8 à 6,5, par exemple par addition d'une base (hydroxyde de sodium) au milieu d'époxydation, comme recommandé dans la demande de brevet WO 99/48882 de la demanderesse (dont le contenu est incorporé par référence dans la présente demande de brevet). Cette base peut être introduite dans un seul réacteur (par exemple, le premier réacteur) ou dans plusieurs réacteurs. Elle est de préférence introduite dans chaque réacteur.

La réaction entre l'oléfine et le composé peroxydé peut s'effectuer en présence d'un sel tel que le chlorure de sodium, comme décrit dans la demande de brevet WO EP99/08703 de la demanderesse (dont le contenu est incorporé par référence dans la présente demande de brevet). Ce sel peut être introduit dans un seul réacteur (par exemple, le premier réacteur) ou dans plusieurs réacteurs. Il est de préférence introduit dans chaque réacteur.

Il peut être avantageux d'introduire l'oléfine à l'état dilué dans un ou plusieurs alcanes. Par exemple, on peut introduire dans les réacteurs d'époxydation un fluide contenant l'oléfine et également au moins 10% (en particulier 20%, par exemple au moins 30 %) en volume d'un ou plusieurs alcanes. Par exemple, dans le cas du propylène, celui-ci peut être mélangé avec au moins 10 % en volume de propane lorsqu'on introduit dans le réacteur le propylène non converti recyclé. Il peut également s'agir d'une source de propylène incomplètement épurée en propane.

Le procédé selon l'invention donne les meilleurs résultats lorsque l'oxiranne est l'oxyde de propylène, l'oléfine est le propylène, le composé peroxydé est le peroxyde d'hydrogène, le solvant est le méthanol et le catalyseur contient du TS-1.

Les exemples qui suivent sont destinés à illustrer la présente invention sans toutefois en limiter la portée.

Les exemples 1 et 2 ont été calculés au moyen du logiciel ASPEN PLUS® de la société ASPEN TECHNOLOGY INC. à l'aide des paramètres cinétiques de la réaction déterminés sur base des essais expérimentaux décrits et des équilibres liquide-vapeur disponibles dans la littérature.

### Exemple 1

Dans cet exemple, la synthèse de l'oxyde de propylène est effectuée dans 2 réacteurs en série avec séparation intermédiaire de l'oxyde de propylène formé au premier réacteur dans une première colonne de rectification. Le mélange soutiré en tête de cette première colonne de rectification, après séparation de la majeure partie du propylène non-converti, est mélangé avec l'effluent du second réacteur et alimenté à une seconde colonne de rectification qui sépare en tête l'oxyde de propylène formé et le solde du propylène et en pied le méthanol, l'eau, le peroxyde d'hydrogène non-converti et les sous-produits ; le pied de cette seconde colonne est envoyé dans une troisième colonne de rectification pour séparer en tête le méthanol et en pied un effluent aqueux.

326.5 kmol/h de peroxyde d'hydrogène accompagné de 1100 kmol/h d'eau sont mélangés à 1500 kmol/h de méthanol, à 600 kmol/h de propylène et à la fraction recyclée du pied de la colonne de rectification du milieu rectionnel quittant le premier étage de réaction sous une pression suffisante pour solubiliser tout le propylène à la température de réaction. Le mélange réactionnel est introduit en continu à 70°C dans un réacteur méthodique contenant 600 kg de catalyseur. Le réacteur est maintenu à 70 °C par un système de réfrigération adéquat.

L'effluent du réacteur est dirigé vers une première colonne de rectification contenant 60 plateaux théoriques (y compris condenseur et bouilleur) ; l'alimentation est effectuée au niveau du 7^{ème} plateau théorique (compté à partir du condenseur) ; la colonne est opérée à 1.1 bar absolu (pression de tête de colonne) ; la température de tête de colonne est maintenue à 40°C (distillat partiellement vaporisé) ; le taux de reflux molaire est fixé à 2 ; le débit total de distillat est ajusté de façon à récupérer dans le distillat 98% de l'oxyde de propylène présent dans l'alimentation.

Le mélange soutiré en pied de cette première colonne, appauvri en oxyde de propylène, est divisé en deux fractions, la première qui contient 90% volume du mélange est recyclée à l'alimentation du premier réacteur ; la seconde qui contient le solde du mélange obtenu en pied de la colonne de rectification est mélangée à 100 kmol/h de propylène à une pression suffisante pour solubiliser l'entièreté du propylène à la température de réaction et introduit en continu à 70 °C dans un second réacteur méthodique contenant 820 kg de catalyseur et maintenu à 70°C par un système de réfrigération adéquat.

Le flux vapeur produit en tête de la première colonne de rectification est envoyé dans un condenseur maintenu à -20°C ; le liquide condensé est séparé de la vapeur, mélangé au distillat liquide produit en tête de la première colonne de rectification et dirigé vers une seconde colonne de rectification contenant 48 plateaux théoriques (y compris condenseur et bouilleur) ce mélange étant alimenté au niveau du 8^{ème} plateau théorique (compté à partir du condenseur) ; l'effluent du second est également dirigé vers cette seconde colonne de rectification ; il est alimenté au niveau du 20^{ème} plateau théorique (compté à partir du condenseur) ; la colonne est opérée à 2.2 bar absolu (pression de tête de colonne) ; le taux de reflux molaire est fixé à 2 ; la température de tête est ajustée de façon à condenser totalement les vapeurs de tête de colonne ; le débit de distillat est ajusté de façon à assurer une teneur de 1 g/kg en oxyde de propylène en pied de colonne.

Le mélange produit en pied de la seconde colonne de rectification est dirigé vers une troisième colonne de rectification contenant 30 plateaux théoriques (y compris condenseur et bouilleur) ; l'alimentation est effectuée au niveau du 15^{ème} plateau théorique (compté à partir du condenseur) ; la colonne est opérée à 1. bar absolu (pression de tête de colonne) la température de tête est ajustée de façon à condenser totalement les vapeurs de tête de colonne ; le taux de reflux massique est fixé à 0.7 ; le débit de distillat est ajusté de façon à assurer une teneur de 1 g/kg en méthanol en pied de colonne. Le distillat produit en tête de la troisième colonne de rectification comprend la majeure partie du méthanol présent dans l'alimentation du premier réacteur ; il est recyclé tel quel à l'alimentation du premier réacteur.

L'effluent produit en pied de la troisième colonne de rectification contient 0.04 kmol/h de peroxyde d'hydrogène non converti, 0.8 kmol/h de méthanol et 13.8 kmol/h de sous-produits (methoxypropanol et propanediol principalement) ; le rendement en C3 atteint 95.7 % pour un taux de conversion du peroxyde d'hydrogène de 99.99%. La consommation d'énergie au bouilleur de la seconde colonne de rectification s'élève à 14000 kW et celle au bouilleur de la troisième colonne à 25300 kW soit une consommation totale pour les 2 colonnes de 39300 kW.

### Exemple 2

Dans cet exemple, la synthèse de l'oxyde de propylène est effectuée dans 2 réacteurs en série avec séparation intermédiaire de l'oxyde de propylène formé au premier réacteur dans une première colonne de rectification. Le mélange soutiré en tête de cette première colonne de rectification, après séparation de majeure partie du propylène non-converti, est alimenté à une seconde colonne de rectification qui sépare en tête l'oxyde de propylène formé et le solde du propylène et en pied le méthanol, l'eau, le peroxyde d'hydrogène et les sous-produits entraînés dans le distillat de la première colonne de rectification ; le pied de cette colonne est recyclé directement au premier réacteur ; l'effluent du second réacteur est envoyé dans une troisième colonne de rectification pour séparer en tête le méthanol et l'oxyde de propylène contenu dans l'alimentation de la colonne et en pied un effluent aqueux ; le mélange produit en tête de la troisième colonne de rectification est recyclée au premier réacteur sans purification supplémentaire.

326.5 kmol/h de peroxyde d'hydrogène accompagné de 1100 kmol/h d'eau sont mélangés à 1500 kmol/h de méthanol, à 600 kmol/h de propylène et à la fraction recyclée du pied de la colonne de rectification du milieu rectionnel quittant le premier étage de réaction sous une pression suffisante pour solubiliser tout le propylène à la température de réaction. Le mélange réactionnel est introduit en continu à 70°C dans un réacteur méthodique contenant 600 kg de catalyseur. Le réacteur est maintenu à 70°C par un système de réfrigération adéquat.

L'effluent du réacteur est dirigé vers une première colonne de rectification contenant 60 plateaux théoriques (y compris condenseur et bouilleur) ; l'alimentation est effectuée au niveau du 7^{ème} plateau théorique (compté à partir du condenseur) ; la colonne est opérée à 1.1 bar absolu (pression de tête de colonne) ; la température de tête de colonne est maintenue à 40°C (distillat partiellement vaporisé) ; le taux de reflux molaire est fixé à 2 ; le débit total de distillat est ajusté de façon à récupérer dans le distillat 98% de l'oxyde de propylène présent dans l'alimentation.

Le mélange soutiré en pied de cette première colonne, appauvri en oxyde de propylène, est divisé en deux fractions, la première qui contient 90% volume du mélange est recyclée à l'alimentation du premier réacteur ; la seconde qui contient le solde du mélange obtenu en pied de la colonne de rectification est mélangée à 100 kmol/h de propylène à une pression suffisante pour solubiliser l'entièreté du propylène à la température de réaction et introduit en continu à 70°C dans un second réacteur méthodique contenant 820 kg de catalyseur et maintenu à 70°C par un système de réfrigération adéquat.

Le flux vapeur produit en tête de la première colonne de rectification est envoyé dans un condenseur maintenu à -20°C ; le liquide condensé est séparé de la vapeur, mélangé au distillat liquide produit en tête de la première colonne de rectification et dirigé vers une seconde colonne de rectification contenant 48 plateaux théoriques (y compris condenseur et bouilleur) ce mélange étant alimenté au niveau du 8^{ème} plateau théorique (compté à partir du condenseur) ; la colonne est opérée à 2.2 bar absolu (pression de tête de colonne) ; le taux de reflux molaire est fixé à 2 ; la température de tête est ajustée de façon à condenser totalement les vapeurs de tête de colonne ; le débit de distillat est ajusté de façon à assurer une teneur de 1 g/kg en oxyde de propylène en pied de colonne.

L'effluent du second réacteur est dirigé vers une troisième colonne de rectification contenant 30 plateaux théoriques (y compris condenseur et bouilleur) ; l'alimentation est effectuée au niveau des 15 plateaux théoriques (compté à partir du condenseur) ; la colonne est opérée à 1. bar absolu (pression de tête de colonne) la température de tête est ajustée de façon à condenser totalement les vapeurs de tête de colonne ; le taux de reflux massique est fixé à 0.7 ; le débit de distillat est ajusté de façon à assurer une teneur de 1 g/kg en méthanol en pied de colonne. Le distillat produit en tête de la troisième colonne de rectification comprend la majeure partie du méthanol et de l'oxyde de propylène présent dans l'alimentation du premier réacteur ; il est recyclé tel quel à l'alimentation du premier réacteur.

L'effluent produit en pied de la troisième colonne de rectification contient 0.02 kmol/h de peroxyde d'hydrogène non converti, 0.8 kmol/h de méthanol et 16.0 kmol/h de sous-produits (methoxypropanol et propanediol principalement) ; le rendement en C3 atteint 95.1 % pour un taux de conversion du peroxyde d'hydrogène de 99.99%. La consommation d'énergie au bouilleur de la seconde colonne de rectification s'élève à 11100 kW et celle au bouilleur de la troisième colonne à 15700 kW soit une consommation totale pour les 2 colonnes de 26800 kW.

## Revendications

1. Procédé continu de fabrication d'oxiranne par réaction d'une oléfine avec un composé peroxydé en présence d'un catalyseur, d'un solvant et de l'eau dans une installation comprenant au moins un réacteur contenant le catalyseur et au moins deux colonnes à distiller, selon lequel :
- on introduit dans le réacteur l'oléfine, le solvant, le composé peroxydé et l'eau,
- on y effectue une époxydation de l'oléfine pour former l'oxiranne,
- on soutire du réacteur un milieu comprenant l'oxiranne formé, l'oléfine non convertie, le solvant, le composé peroxydé non consommé, l'eau et éventuellement des sous-produits,
- on introduit le milieu dans une colonne à distiller (A),
- on recueille en tête de la colonne (A) un mélange contenant la majeure partie de l'oxiranne formé et de l'oléfine non convertie, du solvant, de l'eau, et éventuellement des sous-produits,
- on introduit le mélange dans un condenseur pour éliminer une partie de l'oléfine non convertie,
- on recueille le mélange appauvri en oléfine non convertie sous forme liquide,
- on introduit le mélange liquide dans une deuxième colonne à distiller (B),
- on recueille en pied de la colonne (B) un mélange de solvant et d'eau que l'on recycle dans le réacteur, et
- on recueille en tête de la colonne (B) un milieu à base d'oxiranne.

2. Procédé selon la revendication 1, selon lequel on introduit le milieu recueilli en tête de la colonne (B) dans une troisième colonne à distiller (C), on recueille en tête de la colonne (C) de l'oléfine non convertie que l'on recycle dans le réacteur.

3. Procédé selon la revendication 2, selon lequel on introduit le milieu recueilli en pied de la colonne (C) dans une quatrième colonne à distiller (D), on recueille en tête de la colonne (D) l'oxiranne formé et en pied de la colonne (D) un milieu contenant des sous-produits que l'on recycle dans la colonne (B).

4. Procédé selon l'une quelconque des revendications 1 à 3, selon lequel le réacteur est constitué de deux réacteurs disposés en parallèle, les deux réacteurs pouvant opérer simultanément ou alternativement.

5. Procédé selon l'une quelconque des revendications 1 à 4, selon lequel le milieu soutiré du réacteur est d'abord soumis à une détente avant de l'introduire dans la colonne (A).

6. Procédé selon l'une quelconque des revendications 1 à 5, dans lequel le catalyseur est présent dans le réacteur sous forme de particules dont une partie au moins se trouve à l'état fluidisé.

7. Procédé selon la revendication 6, dans lequel le milieu sortant du réacteur passe à travers un filtre avant d'être introduit dans la colonne (A) ou, le cas échéant, avant d'être soumis à la détente.

8. Procédé selon l'une quelconque des revendications 1 à 7, selon lequel l'installation comporte au moins deux réacteurs disposés en série, dont chacun contient une partie du catalyseur, et au moins trois colonnes à distiller, et selon lequel :
- on introduit dans un premier réacteur une première partie de l'oléfine, le solvant et le composé peroxydé,
- on y effectue une époxydation de la première partie de l'oléfine pour former une première partie de l'oxiranne,
- on soutire de ce premier réacteur un milieu comprenant la première partie d'oxiranne formé, le solvant, l'oléfine non convertie, le composé peroxydé non consommé et éventuellement des sous-produits,
- on introduit le milieu dans une première colonne à distiller (A),
- on recueille en tête de la colonne (A) un premier mélange contenant la majeure partie de l'oxiranne formé et de l'oléfine non convertie, du solvant, de l'eau et éventuellement des sous-produits,
- on recueille en pied de la colonne (A) un premier milieu appauvri en oxiranne contenant du solvant, de l'oléfine non convertie, du composé peroxydé non consommé et éventuellement des sous-produits,
- on introduit dans un deuxième réacteur le premier milieu appauvri en oxiranne, une deuxième partie de l'oléfine et éventuellement une deuxième partie du composé peroxydé,
- on y effectue une époxydation de la deuxième partie de l'oléfine pour former une deuxième partie de l'oxiranne,
- on soutire de ce deuxième réacteur un milieu comprenant la deuxième partie d'oxiranne formé, du solvant, de l'oléfine non convertie, du composé peroxydé non consommé et éventuellement des sous-produits,
- on introduit le milieu dans une autre colonne à distiller (A'),
- on recueille en tête de la colonne (A') un deuxième mélange contenant la majeure partie de l'oxiranne formé et de l'oléfine non convertie, du solvant, de l'eau et éventuellement des sous-produits,
- on recueille en pied de la colonne (A') un deuxième milieu appauvri en oxiranne contenant du solvant, de l'oléfine non convertie, du composé peroxydé non consommé et éventuellement des sous-produits,
- on introduit chacun des premier et deuxième mélanges contenant la majeure partie de l'oxiranne formé et l'oléfine non convertie dans un condenseur pour séparer une partie de l'oléfine non convertie,
- on soutire de chaque condenseur un mélange appauvri en oléfine non convertie sous forme liquide,
- on introduit le mélange liquide dans une deuxième colonne à distiller (B),
- on recueille en pied de la colonne (B) un mélange de solvant et d'eau que l'on recycle dans le premier réacteur, et
- on recueille en tête de la colonne (B) un milieu à base d'oxiranne.

9. Procédé selon la revendication 8, dans lequel l'entièreté du composé peroxydé est introduite dans le premier réacteur.

10. Procédé selon la revendication 8 ou 9, selon lequel :
- on introduit, dans un troisième réacteur, une troisième partie de l'oléfine, le deuxième milieu recueilli en pied de la colonne (A'), et éventuellement une troisième partie du composé peroxydé,
- on y effectue une époxydation de la troisième partie d'oléfine pour former une troisième partie de l'oxiranne,
- on soutire de ce troisième réacteur un milieu comprenant la troisième partie d'oxiranne, le solvant, l'oléfine non convertie, éventuellement le composé peroxydé non consommé et éventuellement des sous-produits,
- on introduit ce milieu dans encore une autre colonne à distiller (A"),
- on recueille en tête de la colonne (A") un milieu que l'on recycle, après condensation, dans le premier réacteur,
- on recueille en pied de la colonne (A") un effluent aqueux.

11. Procédé selon l'une quelconque des revendications 1 à 9, dans lequel l'oxiranne est l'oxyde de propylène, l'oléfine est le propylène, le composé peroxydé est le peroxyde d'hydrogène, le solvant est le méthanol et le catalyseur contient du TS-1.

## Patentansprüche

1. Kontinuierliches Verfahren zur Herstellung von Oxiran durch Umsetzung eines Olefins mit einer Peroxidverbindung in Gegenwart eines Katalysators, eines Lösungsmittels und von Wasser in einer Anlage, die wenigstens einen den Katalysator enthaltenden Reaktor und wenigstens zwei Destillationskolonnen umfaßt, wonach man
- in den Reaktor das Olefin, das Lösungsmittel, die Per-oxidverbindung und das Wasser einführt,
- darin eine Epoxidation des Olefins vornimmt, um das Oxiran auszubilden,
- aus dem Reaktor ein Milieu abnimmt, das das gebildete Oxiran, das nicht umgewandelte Olefin, das Lösungsmittel, die nicht verbrauchte Peroxidverbindung, Wasser und gegebenenfalls Nebenprodukte umfaßt,
- das Milieu in eine Destillationskolonne (A) einführt,
- am Kopf der Kolonne (A) ein Gemisch abnimmt, das den Hauptteil des gebildeten Oxirans und des nicht umgewandelten Olefins, Lösungsmittel, Wasser und gegebenenfalls Nebenprodukte enthält,
- das Gemisch in einen Kondensator einführt, um einen Teil des nicht umgewandelten Olefins abzutrennen,
- das an nicht umgewandeltem Olefin verarmte Gemisch in flüssiger Form gewinnt,
- das flüssige Gemisch in eine zweite Destillationsko-lonne (B) einführt,
- am Fuß der Kolonne (B) ein Gemisch aus Lösungsmittel und Wasser abnimmt, das man zum Reaktor recycliert, und
- am Kopf der Kolonne (B) ein Milieu auf Basis Oxiran gewinnt.

2. Verfahren nach Anspruch 1, wonach man das am Kopf der Kolonne (B) gewonnene Milieu in eine dritte Destillations-kolonne (C) einführt und am Kopf der Kolonne (C) nicht umgewandeltes Olefin gewinnt, das man zum Reaktor recycliert.

3. Verfahren nach Anspruch 2, wonach man das am Fuß der Ko-lonne (C) gewonnene Milieu in eine vierte Destillationskolonne (D) einführt, am Kopf der Kolonne (D) das gebildete Oxiran gewinnt und am Fuß der Kolonne (D) ein Nebenprodukte enthaltendes Milieu abnimmt, das man in die Kolonne (B) recycliert.

4. Verfahren nach einem der Ansprüche 1 bis 3, wonach der Reaktor aus zwei parallel angeordneten Reaktoren besteht, wobei die beiden Reaktoren gleichzeitig oder abwechselnd betrieben werden können.

5. Verfahren nach einem der Ansprüche 1 bis 4, wonach das aus dem Reaktor abgenommene Milieu zunächst einer Entspannung unterworfen wird, bevor es in die Kolonne (A) eingeführt wird.

6. Verfahren nach einem der Ansprüche 1 bis 5, worin der Katalysator im Reaktor in Form von Teilchen vorliegt, von denen sich mindestens ein Teil im fluidisierten Zustand befindet.

7. Verfahren nach Anspruch 6, worin das aus dem Reaktor austretende Milieu durch ein Filter hindurchtritt, bevor es in die Kolonne (A) eingeführt wird oder im gegebenen Falle der Entspannung unterworfen wird.

8. Verfahren nach einem der Ansprüche 1 bis 7, wonach die Anlage wenigstens zwei in Serie angeordnete Reaktoren, von denen jeder einen Katalysatorteil enthält, und wenigstens drei Destillationskolonnen umfaßt, und wonach man
- in einen ersten Reaktor einen ersten Teil des Olefins, das Lösungsmittel und die Peroxidverbindung einbringt,
- darin eine Epoxidation des ersten Teils des Olefins vornimmt, um einen ersten Teil des Oxirans auszubilden,
- aus diesem ersten Reaktor ein Milieu abnimmt, das den ersten Teil des gebildeten Oxirans, das Lösungsmittel, nicht umgewandeltes Olefin, die nicht verbrauchte Per-oxidverbindung und gegebenenfalls Nebenprodukte umfaßt,
- das Milieu in eine erste Destillationskolonne (A) einführt,
- am Kopf der Kolonne (A) ein erstes Gemisch gewinnt, das den Hauptteil des gebildeten Oxirans und des nicht umgewandelten Olefins, Lösungsmittel, Wasser und gegebenenfalls Nebenprodukte enthält,
- am Fuß der Kolonne (A) ein erstes, an Oxiran verarmtes Milieu abnimmt, das Lösungsmittel, nicht umgewandeltes Olefin, nicht verbrauchte Peroxidverbindung und gegebenenfalls Nebenprodukte enthält,
- in einen zweiten Reaktor das erste, an Oxiran verarmte Milieu, einen zweiten Teil des Olefins und gegebenenfalls einen zweiten Teil der Peroxidverbindung einführt,
- darin eine Epoxidation des zweiten Teils des Olefins vornimmt, um einen zweiten Teil des Oxirans auszubilden,
- aus diesem zweiten Reaktor ein Milieu abnimmt, das den zweiten Teil des gebildeten Oxirans, Lösungsmittel, nicht umgewandeltes Olefin, nicht verbrauchte Peroxidverbindung und gegebenenfalls Nebenprodukte umfaßt,
- das Milieu in eine andere Destillationskolonne (A') einführt,
- am Kopf der Kolonne (A') ein zweites Gemisch gewinnt, das den Hauptteil des gebildeten Oxirans und des nicht umgewandelten Olefins, Lösungsmittel, Wasser und gegebenenfalls Nebenprodukte enthält,
- am Fuß der Kolonne (A') ein zweites, an Oxiran verarmtes Milieu gewinnt, das Lösungsmittel, nicht umgewandeltes Olefin, nicht verbrauchte Peroxidverbindung und gegebenenfalls Nebenprodukte enthält,
- jedes der ersten und zweiten Gemische, die den Hauptteil des gebildeten Oxirans und des nicht umgewandelten Olefins enthalten, in einen Kondensator einführt, um einen Teil des nicht umgewandelten Olefins abzutrennen,
- aus jedem Kondensator ein an nicht umgewandeltem Olefin verarmtes Gemisch in flüssiger Form abnimmt,
- das flüssige Gemisch in eine zweite Destillationskolonne (B) einführt,
- am Fuß der Kolonne (B) ein Gemisch aus Lösungsmittel und Wasser gewinnt, das man zum ersten Reaktor recycliert, und
- am Kopf der Kolonne (B) ein Milieu auf Basis Oxiran gewinnt.

9. Verfahren nach Anspruch 8, worin die gesamte Peroxidverbindung in den ersten Reaktor eingeführt wird.

10. Verfahren nach Anspruch 8 oder 9, wonach man
- in einen dritten Reaktor einen dritten Teil des Olefins, das am Fuß der Kolonne (A') gewonnene zweite Milieu und gegebenenfalls einen dritten Teil der Peroxidverbindung einführt,
- darin eine Epoxidation des dritten Teils des Olefins vornimmt, um einen dritten Teil des Oxirans auszubilden,
- aus diesem dritten Reaktor ein Milieu abnimmt, das den dritten Teil des Oxirans, das Lösungsmittel, das nicht umgewandelte Olefin, gegebenenfalls die nicht verbrauchte Peroxidverbindung und gegebenenfalls Nebenprodukte umfaßt,
- dieses Milieu in noch eine weitere Destillationskolonne (A") einführt,
- am Kopf der Kolonne (A") ein Milieu gewinnt, das man nach dem Kondensieren in den ersten Reaktor recycliert,
- am Fuß der Kolonne (A") einen wässerigen Abstrom gewinnt.

11. Verfahren nach einem der Ansprüche 1 bis 9, worin das Ox-iran das Propylenoxid ist, das Olefin das Propylen ist, die Wasserstoffperoxidverbindung das Wasserstoffperoxid ist, das Lösungsmittel das Methanol ist und der Katalysator TS-1 enthält.

## Claims

1. Continuous process for manufacturing oxirane by reacting an olefin with a peroxide compound in the presence of a catalyst, a solvent and water in a plant comprising at least one reactor containing the catalyst and at least two distillation columns, according to which:
- the olefin, the solvent, the peroxide compound and the water are introduced into the reactor,
- an epoxidation of the olefin is carried out to form the oxirane,
- a medium comprising the oxirane formed, the unconverted olefin, the solvent, the unconsumed peroxide compound, the water and possibly by-products is removed from the reactor,
- the medium is introduced into a distillation column (A),
- a mixture containing the majority of the oxirane formed and unconverted olefin, solvent, water and possibly by-products is collected at the top of column (A),
- the mixture is introduced into a condenser to eliminate some of the unconverted olefin,
- the mixture depleted in unconverted olefin is collected in liquid form,
- the liquid mixture is introduced into a second distillation column (B),
- a mixture of solvent and water is collected at the bottom of column (B) and is recycled into the reactor, and
- an oxirane-based medium is collected at the top of column (B).

2. Process according to Claim 1, in which the medium collected at the top of column (B) is introduced into a third distillation column (C), unconverted olefin is collected at the top of column (C) and is recycled into the reactor.

3. Process according to Claim 2, in which the medium collected at the bottom of column (C) is introduced into a fourth distillation column (D), the oxirane formed is collected at the top of column (D) and a medium containing by-products is collected at the bottom of column (D) and recycled into column (B).

4. Process according to any one of Claims 1 to 3, in which the reactor consists of two reactors arranged in parallel, the two reactors possibly being operated simultaneously or alternately.

5. Process according to any one of Claims 1 to 4, in which the medium removed from the reactor is first subjected to a depressurization before being introduced into column (A).

6. Process according to any one of Claims 1 to 5, in which the catalyst is present in the reactor in the form of particles, at least some of which are in fluidized form.

7. Process according to Claim 6, in which the medium leaving the reactor passes through a filter before being introduced into column (A) or, where appropriate, before being subjected to depressurization.

8. Process according to any one of Claims 1 to 7, in which the plant comprises at least two reactor arranged in series, each of which contains some of the catalyst, and at least three distillation columns, and according to which:
- a first portion of the olefin, the solvent and the peroxide compound are introduced into a first reactor,
- an epoxidation of the first portion of the olefin is carried out therein to form a first portion of the oxirane,
- a medium comprising the first portion of oxirane formed, the solvent, the unconverted olefin, the unconsumed peroxide compound and possibly by-products is removed from this first reactor,
- the medium is introduced into a first distillation column (A),
- a first mixture containing the majority of the oxirane formed and unconverted olefin, solvent, water and possibly by-products is collected at the top of column (A),
- a first medium depleted in oxirane, containing solvent, unconverted olefin, unconsumed peroxide compound and possibly by-products, is collected at the bottom of column (A),
- the first medium depleted in oxirane, a second portion of the olefin and optionally a second portion of the peroxide compound are introduced into a second reactor,
- an epoxidation of the second portion of the olefin is carried out therein in order to form a second portion of the oxirane,
- a medium comprising the second portion of oxirane formed, solvent, unconverted olefin, unconsumed peroxide compound and possibly by-products is removed from this second reactor,
- the medium is introduced into another distillation column (A'),
- a second mixture containing the majority of the oxirane formed and unconverted olefin, solvent, water and possibly by-products is collected at the top of column (A'),
- a second medium depleted in oxirane, containing solvent, unconverted olefin, unconsumed peroxide compound and possibly by-products, is collected at the bottom of column (A'),
- each of the first and second mixtures containing the majority of the oxirane formed and the unconverted olefin is introduced into a condenser to separate out some of the unconverted olefin,
- a mixture depleted in unconverted olefin is removed in liquid form from each condenser,
- the liquid mixture is introduced into a second distillation column (B),
- a mixture of solvent and water is collected at the bottom of column (B) and is recycled into the first reactor, and
- an oxirane-based medium is collected at the top of column (B).

9. Process according to Claim 8, in which all of the peroxide compound is introduced into the first reactor.

10. Process according to Claim 8 or 9, in which:
- a third portion of the olefin, the second medium collected at the bottom of column (A') and optionally a third portion of the peroxide compound are introduced into a third reactor,
- an epoxidation of the third portion of olefin is carried out therein in order to form a third portion of the oxirane,
- a medium comprising the third portion of oxirane, the solvent, the unconverted olefin, possibly the unconsumed peroxide compound and possibly by-products is removed from this third reactor,
- this medium is introduced into yet another distillation column (A"),
- a medium is collected at the top of column (A") and is recycled, after condensation, into the first reactor,
- an aqueous effluent is collected at the bottom of column (A").

11. Process according to any one of Claims 1 to 9, in which the oxirane is propylene oxide, the olefin is propylene, the peroxide compound is hydrogen peroxide, the solvent is methanol and the catalyst contains TS-1.
